# Europäisches Patentamt
## European Patent Office
### Office européen des brevets

(11) Publication number: **0 126 817**
**B1**

## EUROPEAN PATENT SPECIFICATION

(45) Date of publication of patent specification: **19.04.89**

(51) Int. Cl.⁴: **C 07 H 3/06, A 61 K 31/735**

(21) Application number: **83303093.5**

(22) Date of filing: **27.05.83**

(54) **Suppressing of graft rejection in organ transplantation.**

(43) Date of publication of application:
**05.12.84 Bulletin 84/49**

(45) Publication of the grant of the patent:
**19.04.89 Bulletin 89/16**

(84) Designated Contracting States:
**AT BE CH DE FR GB IT LI NL SE**

(56) References cited:
**WO-A-80/01875**
**FR-A-2 320 760**
**US-A-4 406 830**

**CHEMICAL ABSTRACTS, vol. 80, no. 7, February 18, 1974, ref. 35741w, page 192, Columbus, Ohio, US. W.J. ESSELMAN et al.: "Glycosphingolipids of membrane fractions from normal and transplanted canine kidney" NATURE, vol. 291, May 28, 1981, pages 334-335. MASATAKA KASAI et al.: "In vivo effect of anti-asialo GM1 antibody on natural killer activity"**

(73) Proprietor: **SHANKSVILLE CORPORATION N.V.**
**Handelskade 8**
**Curacao Netherlands Antilles (NL)**

(72) Inventor: **Fabricius, Hans-Ake**
**Tunibergweg 11**
**D-7814 Breisach (DE)**
Inventor: **Eckart, Ulrich Kottgen**
**Erwinstrasse 39**
**D-7800 Freiburg (DE)**

(74) Representative: **Dixon, Donald Cossar et al**
**Gee & Co. Chancery House Chancery Lane**
**London WC2A 1QU (GB)**

**Description**

This invention relates to the suppression of the cell mediated host versus graft rejection mechanism in transplanting of organs from a donor to a recipient (host). More particularly, this invention relates to the use of chemical blocking agents for the preparation of a medicament which interferes with the ability of the host's cellular immune system to attack a foreign graft organ.

It is well known that a graft from a genetically dissimilar member of the same species (allogeneic graft) can generate a population of killer T cells which are specifically cytotoxic for graft target cells bearing the major histocampatibility (MHC) antigens of the donor. The mechanism for the generation of these cytotoxic T cells (CTC) involves a complex series of reactions on the cellular level which is believed to be initiated by the presentation to, and recognition by, the host's macrophage cells, of the transplantation antigens on the donor graft. The macrophage cells respond by synthesis and release of a first signal (interleukin 1; IL—1). The interleukin 1, in turn, together with a comitogenic blood serum glycoprotein (PHILIP, as discussed in U.S. Patent Application No. 4406830, filed April 17, 1981) activates a paticular sub-population of accessory cells, generally believed to be T helper cells, for production of a second signal (interleukin 2; IL—2; also known as T cell growth factor: TCGF).

It is the second signal which acts on the sub-population of T cells known as T effector cells to effect T cells growth. The T cell blasts are the cytotoxic T cells or killer T cells which specifically recognize the graft cells and effect the graft rejection process.

Gangliosides are known to be effective in certain therapeutic applications as follows: WO—A—80/01875 discloses the therepeutic application of sialo- and asialo- gangliosides for cholera and other acute diarrhoeas, in a formulation which is suitable only for oral administration, such as pellets or tablets. FR—A—2320760 discloses the therapeutic application of an injectable form of sialo-gangliosides for diseases of the CNS, etc.

It has recently been reported that asialo GM1, which is a glycosphingolipid, is a surface component densely expressed on mouse NK (natural killer) cells and further that the natural killing function of the NK cells — which are closely related to cytotoxic T cells and perhaps the NK cells themselves — are destroyed by anti-asialo GM1 antibodies (Masataka Kasai & al.: "A glycolipid on the surface of mouse natural killer cells", Eur. J. Immunol., Vol. 10, pages 175—180 (1980), and Masataka Kasai & al.: "In vivo effect of anti-asialo GM1 antibody on natural killer activity", Nature, Vol. 291, pages 334—335 (May 28, 1981)).

This finding confirms the experimental results and hypotheses of the present inventors, that desialo GM1 is the surface receptor on human T effector cells which directly receives the second signal from interleukin 2 or perhaps from a "blastogenic factor" which in turn causes the modification of the surface receptor to a form which can react with IL—2 or still alternatively which causes the expression on the T effector cell of a new surface receptor which can react with IL—2.

By whichever of these mechanisms the T effector cells are transformed to cytotoxic T cells, the present invention has been completed on the basis of the finding that incorporating desialo GM1 or similar gangliosides in in vitro T cell culture systems containing IL—2 prevents proliferation of T cell blasts in growing T cell lines and/or prevents transformation of T effector cells to T cell blasts. This observation therefore leads to an in vivo action in which there is modification of the cell mediated immunological graft rejection mechanism by gangliosides which are capable of blocking the formation of cytotoxic T cells at the level of the T effector cells without destroying macrophage cells. Accordingly, the present invention is highly advantageous as compared to presently available techniques for transplant protection, particularly by the use of immunosuppressive drugs. Since immunosuppressive drugs such as cortisone derivatives function by killing the macrophage cells, they also inhibit other immunological reactions of the host such as the humoral antibody system. Therefore, such drugs will subject the patient to increased risk of infectious diseases. In addition, since conventional immunosuppressive therapy usually starts at dosage levels which do not entirely kill off the macrophage cells, there will be some production of IL—1 and IL—2 and resulting T cell blast formation. At the time of the graft transplantation therefore, the consequence of IL—2 and T cell blasts will allow the latter to proliferate until the IL—2 is consumed. This proces generally requires several days. In contrast, the immunosuppressive blocking agents of this invention require less than 24 hours to effectively terminate cytotoxic T cell proliferation and to kill off any existing cytotoxic T cells.

Other common immunosuppressive drugs such as azathioprine and methotrexate are used because of their toxicity to dividing cells, and therefore pose the danger of toxicity for cells of the bone marrow and also of the small intestine. Such defect is not present with the ganglioside blocking agents of this invention.

Accordingly, the present invention provides use of one or more gangliosides selected from the group of desialylated gangliosides, for the preparation of a medicament with an action of inhibiting cell mediated rejection of organ transplants from a donor to a host.

The present invention further provides a medicament with an action of inhibiting cell mediated rejection of organ transplants from a donor to a host and comprising an injectable solution of one or more desialylated gangliosides.

The transplantation of organ grafts such as kidney, heart, liver, pancreatic islets, bone marrow and the like, even when donor and recipient are carefully tissue type matched, inevitably involves the host versus graft (and, in the case of bone marrow transplantation, graft versus host) immunological cell mediated rejection mechanism. The rejection mechanism actually involves several potential pathways, (see for example "Essential immunology", 3rd Edition by Ivan Roitt, Blackwell Scientific Publications (1977), pages 234—239), but the direct killing by sensitized T cells, i.e. cytotoxic T cells, is clearly the most important.

Under normal circumstances, T effector cells are circulating in the blood system. When an organ is transplanted in the host, the host's macrophage cells coming into contact with the graft recognize it as foreign and, after a few days, become sensitized. This sensitization results in the production by the macrophages of interleukin 1 which in turn, with the comitogenic factor PHILIP induces cellular production of interleukin 2. IL—2 and probably also a blastogenic factor which is itself synthesized by either the sensitized macrophage cells or by an accessory cell, probably a T cell, activate resting T effector cells to form T cell blasts, i.e. cytotoxic T cells. If nothing is done to prevent the formation of the cytotoxic T cells, they will attack and kill the graft cells until the entire graft is destroyed.

Therefore, to prevent the T effector cells from undergoing blastogenesis to their activated natural killing form, the T effector cell must somehow be prevented from receiving the signal from IL—2 and/or the blastogenic fctor. Since it is known that the relevant surface receptor on the T effector cell is desialo GM1, either or both of the IL—2 or blastogenic factor must be capable of reacting with this ganglioside.

Accordingly, the effect of desialo GM1 or structurally similar ganglioside in vivo is that all or nearly all of the soluble mediators which could otherwise react with the desialo GM1 surface receptor will instead react with the introduced desialo GM1 blocking agent. The blastogenic signal is thereby prevented from activating the T effector cell and no blast transformation takes place.

Other blocking agents which can be used, in addition to desialo GM1 include other desialylated gangliosides such as desialo GD and its subgroups, especially GD1a and GD1b, and GT and its subgroups, especially GT1.

Desialo (or asialo) GM1 has the formula: Gal(beta 1—3)GA1NAc(beta 1—4)Glc(beta 1—1)Cer. The formula of the gangliosides GD1a, GD1b and GT1 are described on page 106 in "Cell surface carbohydrate chemistry" edited by R. E. Harmon, Academic Press Inc. (1978), chapter by L. D. Kohn & al. The definition of these abbreviations are provided, for example, in "The Nomenclature of lipids", Eur. J. Biochem. 79, 11 (1977). Desialo GM1 and the other useful ganglioside blocking agents are commercially available in highly purified form.

The gangliosides are characterized by the presence of a portion which is soluble in organic solvents, and a portion which is soluble in aqueous solvents. Accordingly, the gangliosides can be dissolved in an aqueous/organic solvent mixture or coupled to water-soluble organic carriers. Suitable organic carriers would include, for example, albumin, antibodies, lectins, glycoproteins, desoxyribonucleic acid, dextrans and liposomes. Techniques for the use of these organic carriers can be found in "Drug Carriers in Biology and Medicine" edited by G. Gregoriadis, Academic Press, (1979). Suitable organic solvent would include for example alcohol and dimethylsulfoxide. Organic solvent to water ratios from about 0:100 to about 1:100 can be used.

The effective amount of ganglioside blocking agent is that which is sufficient to react with the soluble mediators of the T cell blast transformation which would be available for reaction with the desialo GM1 surface receptors. Generally, amounts of blocking agent in the range from about 0.5 µg to 10 mg per kilogram of body weight per day will be sufficient to effectively suppress T cell blastogenesis and T cell proliferation.

The effective daily dosage can be in an amount suitable for a single injection or as two or more injections. However, since it is most probable that the ganglioside is reacting with interleukin 2, a single daily injection should be sufficient because about a 24 hour period is required for maximal production of IL—2. Each injection can be administered intravenously. In order to minimize the risk of infections after the operation, it seems convenient not to start the treatment with the blocking agent until about two days after the patient has received the graft. It is well established that the rejection mechanism will not be activated until at least two days after the operation.

The present invention can prevent rejection of a graft of an organ from a donor to a host for any degree of relationship, i.e. siblings; parent-child; tissue typed but unrelated, etc. In general the principle applications presently contemplated include heart, kidney, liver, pancreatic islets, and bone marrow transplants.

The present invention is further illustrated by the following non-limiting Examples:

## Example 1

The following test is conducted to determine the inhibition of tumor cell killing capability of cytotoxic T cells activated by interleukin 2. Tumor cells from HeLa strain are labelled with radioactive chromium. The cells are washed and divided into two portions comprising the same cell number. Both portions are coincubated with cytotoxic T lymphoblasts for 18 hours. The culture medium contains IL—2. To one of the portions, at the beginning of the cocultivation, desialo GM1 is added in concentration of 2 micrograms per ml of culture medium. The culture medium is decanted and the cells are pelleted in a centrifuge. The release of chromium from the labelled tumor cells indicate that the cells have been killed. The results are summarized in Table 1.

TABLE 1

| | | % of Cr released | |
| | Target and T cell | Target and Control | Target and IL—2 |
| --- | --- | --- | --- |
| without asialo GM1 | 85.1 | 4.1 | 0.1 |
| with asialo GM1 | 5.3 | 3.8 | — |

Example 2

This Example illustrates that asialo GM1 reacts with a soluble mediator for T lymphocyte blastogenesis inhibiting cytotoxic T cell formation. Peripheral blood lymphocytes are isolated by a density gradient centrifugation on ficoll hypaque and incubated 24 hours in the absence of serum proteins.

Interleukin 2 containing tissue culture medium RPMI 1640 is treated with asialo GM1 in a concentration of 5 micrograms per ml of culture medium. The incubation with asialo GM1 is performed at 37°C for 37 minutes. Thereafter, the treated culture medium is dialysed against RPMI 1640 without supplements for 24 hours at a temperature of 4°C. The cells are incubated in the dialysed (and thus containing no free asialo GM1) culture medium or 7 days. For dialysis, the critical micellar concentration (CMC) of the ganglioside has to be regarded. If it is exceeded, dialysis will be impossible. CMC values for each ganglioside have been established, and are readily available. In contrast to the situation in controls not treated with asialo GM1, no blast transformation of T cells is observed.

**Claims**

1. Use of one or more gangliosides selected from the group of desialylated gangliosides, for the preparation of a medicament with an action of inhibiting cell mediated rejection of organ transplants from a donor to a host.

2. Use as claimed in claim 1, wherein the desialylated gangliosides are asialo GM1, GD1a, GD1b and GT1.

3. A medicament with an action of inhibiting cell mediated rejection of organ transplants from a donor to a host and comprising an injectable solution of one or more desialylated gangliosides.

4. Use as claimed in claim 1 or 2 or a medicament as claimed in claim 3 in which the medicament is in the form of an injectable solution of the one or more gangliosides in an aqueous organic solvent mixture having a ratio of organic solvent to water of 0:100 to 1:100.

5. Use or medicament as claimed in claim 4, in which said aqueous organic solvent mixture is an alcohol/water mixture.

6. Use as claimed in claim 1 or 2 or a medicament as claimed in claim 4, in which the medicament is in the form of an injectable aqueous solution of a complex of the one or more gangliosides with a water soluble organic carrier.

7. Use or medicament as claimed in claim 6, in which said organic carrier is selected from albumin, antibodies, lectins, glycoproteins, desoxyribonucleic acid, dextran and liposomes.

8. Use as claimed in claim 1 or 2 or a medicament as claimed in any of claims 3 to 7, in which the medicament is in a form for administration to a host at a dosage of from 0.5 µg to 10 mg/kg body wt/day.

**Patentansprüche**

1. Verwendung von einem oder mehreren aus der Gruppe der desialylierten Ganglioside ausgewählten Ganglioside zur Herstellung eines Medikamentes mit einer hemmenden Wirkung auf die durch Zellen verursachte Abstossung eines in einen Empfänger transplantierten Spenderorganes.

2. Verwendung nach Anspruch 1, bei der die desialylierten Ganglioside Asialo-GM1, -GD1a, -GD1b und -GT1 sind.

3. Eine Medikament mit einer hemmenden Wirkung auf die durch Zellen verursachte Abstossung eines in einen Empfänger transplantierten Spenderorganes und umfassend eine injizierbare Lösung aus einem oder mehreren desialylierten Gangliosiden.

4. Verwendung nach Anspruch 1 oder 2 oder ein Medikament nach Anspruch 3, wobei das Medikament die Form einer injizierbaren Lösung bestehend aus dem einen oder mehreren Gangliosiden in einem wässrig-organischen Lösungsmittelgemisch aufweist, wobei das Verhältnis von organischem Lösungsmittel zu Wasser zwischen 0:100 und 1:100 liegt.

5. Verwendung oder Medikament nach Anspruch 4, wobei das besagte wässrig-organische Lösungsmittelgemisch ein Alkohol/Wassergemisch ist.

6. Verwendung nach Anspruch 1 oder 2 oder ein Medikament nach Anspruch 4, wobei das Medikament die Form einer injizierbaren wässrigen Lösung aus einem Komplex aus dem einen oder mehreren Gangliosiden mit einem wasserlöslichen organischen Träger aufweist.

7. Verwendung oder Medikament nach Anspruch 6, wobei der genannte organische Träger aus Albumin, Antikörpern, Lectinen, Glykoproteinen, Desoxyribonuleinsäure, Dextran und Liposomen.

8. Verwendung nach Anspruch 1 oder 2 oder Medikament nach irgendeinem der Anspruche 3 bis 7, wobei dad Medikament in einer Form ist, die einem Empfänger in einer Dosierung von 0.5 µg bis 10 mg/kg Körpergewicht/Tag verabreicht werden kann.

## Revendications

1. Utilisation d'un ou plusieurs gangliosides choisis dans la classe formée par les gangliosides désialylés pour la préparation d'un médicament ayant une action inhibitrice du rejet à médiation cellulaire d'organes transplantés d'un donneur à un hôte.

2. Utilisation suivant la revendication 1, dans laquelle les gangliosides désialylés sont asialo-GM1, -GD1a, -GD1b et -GT1.

3. Médicament ayant une action inhibitrice du rejet à médiation cellulaire d'organes transplantés d'un donneur à un hôte et comprenant une solution injectable d'un ou plusieurs gangliosides désialylés.

4. Utilisation suivant la revendication 1 ou 2 ou médicament suivant la revendication 3, dans lesquels le médicament est sous la forme d'une solution injectable du ou des gangliosides dans un mélange aqueux de solvant organique ayant un rapport du solvant organique à l'eau de 0:100 à 1:100.

5. Utilisation ou médicament suivant la revendication 4, dans lesquels le mélange aqueux de solvant organique est un mélange alcool/eau.

6. Utilisation suivant la revendication 1 ou 2 ou médicament suivant la revendication 4, dans lesquels le médicament est sous la forme d'une solution aqueuse injectable d'un complexe du ou des gangliosides avec un support organique hydrosoluble.

7. Utilisation ou médicament suivant la revendication 6, dans lesquels le support organique est choisi parmi l'albumine, les anticorps, les lectines, les glycoprotéines, l'acide désoxyribonucléique, le dextrane et les liposomes.

8. Utilisation suivant la revendication 1 ou 2 ou médicament suivant l'une quelconque des revendications 3 à 7, dans lesquels le médicament est sous une forme pour l'administration à un hôte en une dose de 0,5 µg à 10 mg/kg de poids du corps/jour.